# EUROPÄISCHE PATENTSCHRIFT

(11) **EP 1 598 034 B1**
(45) Veröffentlichungstag und Bekanntmachung des Hinweises auf die Patenterteilung: **30.03.2011**
(21) Anmeldenummer: 04011960.4
(22) Anmeldetag: 19.05.2004
(51) Int. Cl.: A61F 2/40

(54) **Glenoidverankerung**
Glenoidal anchorage
Ancrage glénoIde

(43) Veröffentlichungstag der Anmeldung: 23.11.2005
(73) Patentinhaber: Zimmer GmbH, 8404 Winterthur (CH)
(72) Erfinder: Baum, Ines, 78467 Konstanz (DE); Rauscher, Markus, 6319 Allenwinden (CH); Wendt, Peter, 8542 Wiesendangen (CH)
(74) Vertreter: Manitz, Finsterwald & Partner GbR

(56) Entgegenhaltungen:
- EP-A- 0 339 530
- FR-A- 2 704 747
- FR-A- 2 780 635
- US-A1- 2003 114 933

## Beschreibung

Die Erfindung betrifft eine Gelenkverbindung für eine Schulterendoprothese mit einer Glenoidverankerung für ein Gelenkteil und einem an der Glenoidverankerung befestigbaren, halbkugelförmigen Gelenkteil. Die Glenoidverankerung umfasst eine am resezierten Glenoidknochen aufsetzbare Platte, die Bohrungen für Befestigungselemente zur Verankerung im Glenoidknochen aufweist, und einen Stumpf zur Befestigung des Gelenkteils, wobei der Stumpf mit der Platte fest verbunden ist. Eine derartige Gelenkverbindung ist aus der US 2003/0114933 A1 bekannt.

Künstliche Schultergelenke werden beispielsweise bei einer Arthrose mit fortgeschrittenem Verschleiß der Gelenkflächen des Schultergelenks oder bei komplexen Oberarmkopffrakturen eingesetzt. Bei einer Totalendoprothese werden die Gelenkflächen sowohl des Oberarmkopfs als auch der Gelenkpfanne des Glenoids ersetzt. Bei geschädigter Rotatorenmanschette oder fehlgeschlagener Versorgung mit einer konventionellen Schulterprothese kann eine inverse Schulterendoprothese verwendet werden, wobei der künstliche Prothesenkopf und die künstliche Kugelpfanne bezüglich ihrer Positionen zu einem natürlichen Schultergelenk vertauscht sind.

Zur Befestigung der künstlichen Kugelpfanne oder des künstlichen Prothesenkopfs bei einer konventionellen bzw. inversen Schulterendoprothese am Glenoid ist üblicherweise eine separate Glenoidverankerung der eingangs genannten Art vorgesehen, die mittels Knochenschrauben im Glenoid verankerbar ist. Das jeweilige glenoidseitig anzubringende Gelenkteil, Kugelpfanne oder Prothesenkopf, kann dann wiederum auf die Platte aufgesetzt und mittels einer Schraubverbindung an einer derartigen Glenoidverankerung befestigt werden. Hierzu weist der Stumpf der bekannten Glenoidverankerung ein Innengewinde und ein Zapfen des anzubringenden Gelenkteils ein entsprechendes Aussengwinde auf.

Nachteilig bei einer derartigen Glenoidverankerung ist jedoch, dass bei einer Schraubverbindung eine frühzeitige Lockerung der Verbindung des Gelenkteils zu der Glenoidverankerung auftreten kann. Insbesondere können bei einer Schraubverbindung Partikel oder Späne abgerieben werden, die zu Entzündungen führen und/oder die vorstehend erwähnte frühzeitige Lockerung verursachen können.

Der Erfindung liegt die Aufgabe zugrunde, eine Gelenkverbindung mit einer Glenoidverankerung der eingangs genannten Art zu schaffen, die eine sichere Verankerung im Glenoid und zugleich eine zuverlässige Verbindung mit einem Gelenkteil einer Schulterendoprothese gewährleistet.

Die Lösung dieser Aufgabe erfolgt durch die Merkmale des Anspruchs 1 und insbesondere dadurch, dass der Stumpf auf seiner Innenseite eine Aufnahme mit einer konischen Fläche für einen Press-Sitz mit einem konischen Zapfen des Gelenkteils aufweist.

Die Erfindung wie in Patentanspruch 1 dargestellt zeichnet sich insbesondere dadurch aus, dass in dem Stumpf eine konische Aufnahme, d.h. ein Innenkonus ausgebildet ist, in den ein Zapfen eines Gelenkteils eingreifen kann, der ebenfalls konisch ausgebildet ist. Hierdurch kann zwischen der Glenoidverankerung und dem Gelenkteil ein Press-Sitz geschaffen werden, der eine besonders feste und stabile Verbindung gewährleistet, wobei insbesondere die Gefahr eines Partikelabriebs und/oder einer frühzeitigen Lockerung verringert ist. Die Glenoidverankerung besitzt weiterhin den Vorteil, dass die konische Aufnahme des Stumpfs und der konische Zapfen des Gelenkteils besonders einfach hergestellt werden können. Insbesondere können Stumpf und Zapfen aus verschiedenen Materialien hergestellt werden, ohne dass eine erhöhte, durch verschiedene Härtegrade der Materialien bedingte Gefahr eines Partikelabriebs des weicheren Materials besteht. Platte und Stumpf sind bevorzugt jeweils aus Metall gefertigt.

Darüber hinaus kann erfindungsgemäß die Konusverbindung zwischen Stumpf und Zapfen besonders einfach geschaffen werden, beispielsweise durch einen einzigen Schlag mit definierter Schlagkraft, insbesondere mittels eines Einschlägers, wobei im Gegensatz zu einer Schraubverbindung bei einer konischen Verbindung eine gleichmäßige Verspannung entlang der gesamten Innenseite des Stumpfs erreicht werden kann.

Vorteilhafte Ausführungsformen der Erfindung sind auch in den Unteransprüchen, der Beschreibung sowie der Zeichnung angegeben.

Die Aufnahme der Glenoidverankerung besitzt bevorzugt einen ganzen Konuswinkel zwischen 3° und 5°, in die der konische Zapfen des Gelenkteils mit identischem Konuswinkel unter Ausbildung des Press-Sitzes eingreifen kann. Ferner ist bevorzugt ein mittlerer Konusdurchmesser zwischen 5 und 8 mm vorgesehen. Derartige Konuswinkel und - durchmesser haben sich im Hinblick auf die beschränkten Verankerungsmöglichkeiten am Glenoidknochen als besonders geeignet erwiesen und gewährleisten zugleich einen besonders starken Halt des Press-Sitzes.

Die mittlere Wandstärke des Stumpfes beträgt bevorzugt zwischen 0,3 und 2 mm. Bei einer derartigen mittleren Wandstärke, die ebenfalls den beschränkten Verankerungsmöglichkeiten am Glenoidknochen Rechnung trägt und sich als besonders geeignet herausgestellt hat, kann der Stumpf, der bevorzugt zum Glenoidknochen hin von der Platte absteht, zur zusätzlichen Verankerung der Glenoidverankerung im Glenoidknochen dienen, da beim Einsetzen bzw. Einschlagen des Zapfens des Gelenkteils zur Ausbildung der Konusverbindung der Stumpfdurchmesser des Stumpfs zumindest geringfügig geweitet und somit eine Verankerung des Stumpfs im Glenoidknochen erreicht werden kann.

Des Weiteren wird erfindungsgemäß vorgeschlagen, dass die Platte zum Glenoidknochen hin gewölbt ist. Die Platte wird bevorzugt im Bereich der natürlichen Schulterpfanne im resezierten Glenoidknochen verankert. Bei einer zum Glenoidknochen hin gewölbten Platte, die der natürlichen Wölbung der Schulterpfanne nachempfunden ist, kann der Knochenabtrag am zu resezierenden Glenoidknochen verringert werden. Ferner kann eine gewölbte Platte, die an einem entsprechend geformten resezierten Glenoidknochen aufgesetzt wird, aufgrund der Wölbung sicherer am Glenoidknochen verankert werden als eine ebene Platte, die auf eine ebene Stelle des Glenoidknochens aufgesetzt wird.

Nach einer bevorzugten Ausführungsform der Erfindung ist zur Ausbildung der Wölbung die Platte mit einer Außenfläche und mit einer Innenfläche versehen, welche Ausschnitte von Kugelflächen mit einem Radius zwischen 22 und 40 mm sind. Die Mittelpunkte der beiden Kugelflächen können voneinander beabstandet sein und die Radien der Außen- und der Innenfläche können voneinander abweichen.

Es ist weiterhin bevorzugt, dass bei einer ovalen Platte die in Längsrichtung abschließenden Kanten der Platte einen Abstand zwischen 25 und 50 mm aufweisen. Unter einer ovalen Platte ist jede Platte zu verstehen, die bei Vernachlässigung der Wölbung der Platte von einer kreisförmigen Grundform abweicht. Hierunter fallen beispielsweise eine ellipsenförmige, eiförmige oder länglich runde Platte oder jede beliebige andere Platte, der eine Längsrichtung zuweisbar ist. Der Abstand der in Längsrichtung der Platte ausgebildeten Kanten definiert dabei die maximale Erstreckung der Platte.

Nach einer besonderen Ausgestaltung der Erfindung ist der Stumpf exzentrisch an der Platte angebracht, wobei die Platte beispielsweise jede vorstehend erläuterte Form oder jede beliebige andere Form, der keine Längsrichtung zuweisbar ist, aufweisen kann. Durch die Ausbildung des Stumpfs außerhalb eines Mittelpunkts oder einer Polachse der Platte kann die Glenoidverankerung besonders vorteilhaft an die natürliche Geometrie des Glenoidknochens und/oder auf die beschränkten Verankerungsmöglichkeiten am Glenoidknochen angepasst werden.

Vorzugsweise ist der Schnittpunkt der Längsachse des Stumpfs mit einer Verbindungsgeraden von Kanten der Platte um einen Betrag von 2 bis 8 mm von der Mitte der Verbindungsgeraden zu einer unteren Kante hin verschoben. Des Weiteren kann die Längsachse des Stumpfs gegenüber einer Senkrechten zu einer Verbindungsgeraden von Kanten der Platte um einen Winkel β von 3° bis 7° nach unten geneigt ist. Bei den Kanten handelt es sich bevorzugt jeweils um die in Längsrichtung abschließenden Kanten einer ovalen Platte, wobei die Längsrichtung insbesondere superior-inferior orientiert ist.

Besonders vorteilhaft ist es, wenn die Längsachse der konischen Fläche mit der Längsachse des Stumpfs zusammenfällt. Hierdurch wird eine symmetrische Ausbildung des Press-Sitzes erreicht, die eine besonders zuverlässige Befestigung des Gelenkteils an der Glenoidverankerung gewährleisten kann. Dies bedeutet insbesondere, dass die Längsachse des Zapfens des Gelenkteils ebenfalls mit der Längsachse des Stumpfs zusammenfällt.

Nach einer weiteren bevorzugten Ausführungsform der Erfindung weist der Stumpf zur drehsicheren Verankerung an seiner Außenseite mehrere insbesondere messerartige Längsrippen auf. Die Längsrippen verlaufen dabei im Wesentlichen parallel zur Längsachse des Stumpfs und stehen finnenartig von diesem radial nach außen ab. Die Längsrippen erstrecken sich dabei bevorzugt über die gesamte Höhe des Stumpfs und können sich beim Verankern der Glenoidverankerung in den Glenoidknochen einschneiden, um einer möglichen Verdrehung der Glenoidverankerung entgegenzuwirken.

Ferner können erfindungsgemäß von der Platte zum Glenoidknochen hin mehrere insbesondere ringförmige Schneiden mit Durchbrüchen für das Einwachsen von Knochenmaterial vorstehen. Die ringförmigen Schneiden sind bevorzugt durch Einpressen in eigens dafür vorgesehene Bohrungen an der Platte angebracht. Durch in die Durchbrüche eingewachsenes Knochenmaterial kann die Festigkeit der Verankerung der Glenoidverankerung am Glenoidknochen zusätzlich erhöht werden.

Zur Realisierung einer inversen Schulterendoprothese ist das Gelenkteil halbkugelförmig ausgebildet und schließt mit einer oberen Kante der Platte bündig ab oder steht nur geringfügig über diese vor und steht über eine untere Kante der Platte weiter vor als über die obere Kante, wobei bevorzugt die obere Kante superior und die untere Kante inferior angeordnet ist. Hierdurch kann einer auf der Kugelfläche des Gelenkteils gleitenden und am Humerus befestigbaren kugelförmigen Lagerschale oder Kugelpfanne auf der unteren Seite des Gelenkteils ein größerer Schwenkbereich ermöglicht werden. Insbesondere kann hierdurch ein Anstoßen des Humerus am Glenoidknochen verhindert werden.

Bevorzugt sind verschiedene halbkugelförmige Gelenkteile mit unterschiedlichen Kugeldurchmessern vorgesehen. Der Operateur kann hierbei aus einem Satz voneinander verschiedener Gelenkteile dasjenige Gelenkteil auswählen, das hinsichtlich des Kugeldurchmessers für den jeweiligen Patienten am besten geeignet erscheint.

Vorzugsweise ist die Platte in einer der von einer Kreisform abweichenden, insbesondere ovalen Außenkontur der Platte entsprechenden Ausnehmung des halbkugelförmigen Gelenkteils versenkbar. Die insbesondere ovale Platte ist mit ihrer Außenkontur in der passenden Ausnehmung des halbkugelförmigen Gelenkteils versenkbar, um die Winkellage der Platte zum halbkugelförmigen Gelenkteil festzulegen, wenn der Press-Sitz zwischen dem Zapfen des Gelenkteils und der konischen Fläche der Aufnahme des Stumpfs erfolgt.

Die Platte und der Stumpf können aus einem besonders körperverträglichen Material, insbesondere Titan oder einer Titanlegierung und das halbkugelförmige Gelenkteil aus einem verschleißfesten Werkstoff, beispielsweise aus einer Cr-Mo-Legierung, der zusätzlich Ta beigemischt sein kann, bestehen.

Insbesondere kann das halbkugelförmige Gelenkteil aus einer Cr-Mo-Legierung bestehen und mit einer am Humerus befestigbaren kugelförmigen Lagerschale, die aus einer Cr-Mo-Legierung besteht, gepaart sein. Eine inverse Schulterendoprothese ist üblicherweise derart ausgebildet, dass das halbkugelförmige Gelenkteil und die kugelförmige Lagerschale exakt ineinander greifen, wobei im Gegensatz zu einem natürlichen Schultergelenk oder einer konventionellen Schulterendoprothese keine Verschiebung des Kugelkopfs in der Pfanne erlaubt ist, um eine Subluxation zu verhindern. Bei einer inversen Schulterendoprothese können deshalb auch zwei Bauteile, die jeweils aus einer Cr-Mo-Legierung bestehen, miteinander gepaart werden. Grundsätzlich kann die kugelförmige Lagerschale jedoch auch aus einem Kunststoff, beispielsweise Polyethylen, und das halbkugelförmige Gelenkteil wegen der Konusverbindung aus Keramik bestehen.

Zur Realisierung einer konventionellen Schulterendoprothese kann ein schalenförmiges Gelenkteil mit einer insbesondere der Außenkontur der Platte entsprechenden Kontur an der Platte befestigbar sein. In das schalenförmige Gelenkteil, das bevorzugt aus einem Kunststoff, beispielsweise aus Polyethylen besteht, kann dann ein an dem Humerus angebrachter Gelenkkopf eingreifen.

Die Erfindung wird im Folgenden beispielhaft unter Bezugnahme auf die Zeichnung beschrieben. Es zeigen:
- Fig. 1: eine perspektivische Ansicht einer Glenoidverankerung mit einer Platte und einem Stumpf,
- Fig. 2a, b eine: Frontal- bzw. Seitenansicht der Glenoidverankerung von Fig. 1,
- Fig. 3: einen Querschnitt durch die Glenoidverankerung von Fig. 2a entlang der Linie IV-IV,
- Fig. 4: eine vergrößerte Darstellung einer an der Platte angebrach- ten ringförmigen Schneide mit Durchbrüchen,
- Fig. 5: einen Querschnitt durch die Glenoidverankerung von Fig. 2a,
- Fig. 6a - d: verschiedene Ansichten und einen Querschnitt eines halb- kugelförmigen Gelenkteils mit einem ersten Kugeldurch- messer,
- Fig. 7a - d: verschiedene Ansichten und einen Querschnitt eines halb- kugelförmigen Gelenkteils mit einem zweiten Kugeldurch- messer,
- Fig. 8a - c: eine erfindungsgemäße Gelenkverbindung mit der Glenoid- verankerung nach den Fig. 1 bis 5 und dem halbkugelför- migen Gelenkteil nach Fig. 6 bzw. Fig. 7 im zusammenge- setzten Zustand, und
- Fig. 9: eine Gelenkverbindung mit der Glenoidverankerung nach den Fig. 1 bis 5 und einem schalenförmigen Gelenkteil im zusammengesetzten Zustand.

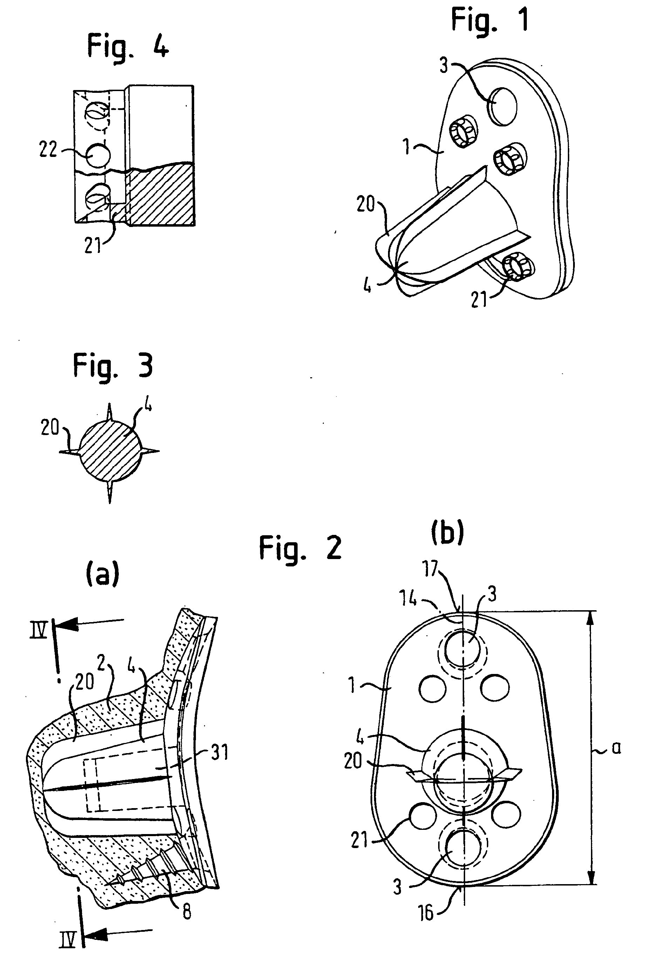

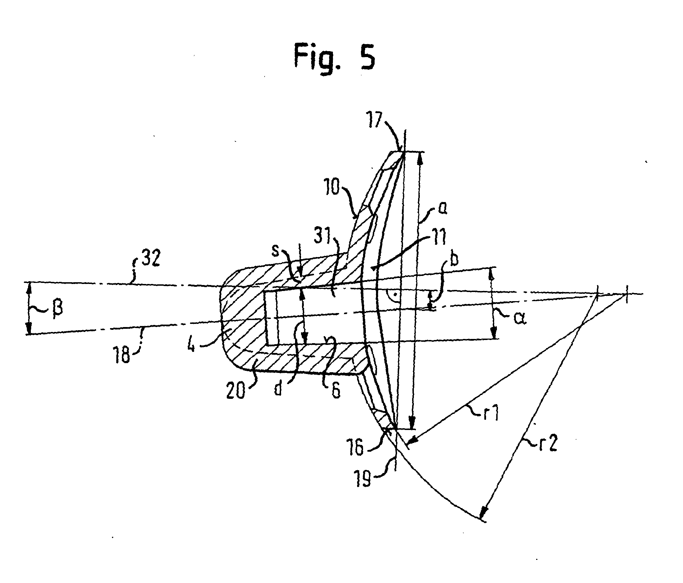

Die Fig. 1 bis 5 zeigen eine Glenoidverankerung für ein an anderer Stelle näher erläutertes Gelenkteil mit einer metallischen Platte 1, die an einem resezierten Glenoidknochen 2 aufgesetzt ist, wobei der Glenoidknochen 2 in Fig. 2a der Einfachheit halber im Querschnitt dargestellt ist. In der metallischen Platte 1 sind zwei Bohrungen 3 für jeweils eine Knochenschraube 8 vorgesehen, die jeweils der Verankerung der Glenoidverankerung im Glenoidknochen 2 dienen, wobei in Fig. 2a lediglich eine Knochenschraube 8 dargestellt ist.

Die aus Titan oder einer Titanlegierung gefertigte Platte 1, die spiegelsymmetrisch zu einer Spiegelebene ausgebildet ist, besitzt in der Frontalansicht von Fig. 2b eine ovale Grundform, durch die eine Längsrichtung 14 der Platte 1 definiert wird. Die beiden Bohrungen 3 sind im Bereich der in Längsrichtung 14 gelegenen Enden der ovalen Platte 1 angeordnet, die durch eine obere Kante 17 und eine untere Kante 16 abgeschlossen werden. Die die Platte 1 in Längsrichtung 14 abschließenden Kanten 16, 17 besitzen einen gegenseitigen Abstand a, der zwischen 25 und 50 mm liegen kann.

Die Platte 1 ist ferner zum Glenoidknochen 2 hin gewölbt. Die Platte 1 besitzt eine Außenfläche 10, die dem Glenoidknochen 2 zugewandt ist, und eine Innenfläche 11, die vom Glenoidknochen 2 abgewandt ist. Zur Ausbildung der Wölbung der Platte 1 sind die Außenfläche 10 und die Innenfläche 11 jeweils als Ausschnitte oder Abschnitte von Kugelflächen ausgelegt. Die Radien r1, r2 der der Außen- und der Innenfläche 10, 11 zugeordneten Kugelflächen, die zwischen 22 und 40 mm liegen können, weichen geringfügig voneinander ab, wobei zusätzlich die Mittelpunkte der beiden Kugelflächen gegeneinander verschoben sind.

An der Außenfläche 10 der Platte 1 sind weiterhin mehrere ringförmige Schneiden 21 angebracht, die in Richtung des Glenoidknochens 2 von der Platte 1 abstehen. Die ringförmigen Schneiden 21 weisen jeweils eine Vielzahl von Durchbrüchen 22 auf, in die nach der Verankerung der Glenoidverankerung im Glenoidknochen 2 Knochenmaterial einwächst, um die Festigkeit der Verankerung der Glenoidverankerung zu erhöhen.

Die Glenoidverankerung umfasst ferner einen fingerhutartigen Stumpf 4, der fest mit der Platte 1 verbunden und exzentrisch an dieser angebracht ist. Der Stumpf 4, der aus Titan oder einer Titanlegierung gefertigt ist, steht in Richtung des Glenoidknochens 2 von der ovalen Platte 1 ab und weist an seiner konischen Außenseite mehrere messerartige Längsrippen 20 auf, die im Wesentlichen parallel zu einer Längsachse 18 des Stumpfs 4 orientiert sind und die Glenoidverankerung gegen eine Drehung um die Längsachse 18 des Stumpfs 4 sichern.

Zwischen der oberen Kante 16 und der unteren Kante 17 der Platte 1 ist eine Verbindungsgerade 19 definiert. Die Längsachse 18 des Stumpfs 4 ist gegenüber einer Senkrechten 32 zu der Verbindungsgeraden 19 um einen Neigungswinkel β, der 3° bis 7° betragen kann, nach unten geneigt. Der Schnittpunkt der Längsachse 18 des Stumpfs mit der Verbindungsgeraden 19 der beiden Kanten 16, 17 ist dabei um einen Betrag b, der 2 bis 8 mm betragen kann, von der Mitte der Verbindungsgeraden 19 in Richtung der unteren Kante 16 der Platte 1 verschoben.

Der Stumpf 4 weist auf seiner Innenseite eine Aufnahme 31 mit einer konischen Fläche 6 auf. Die Längsachse 18 der konischen Fläche 6 fällt dabei mit der Längsachse 18 der Stumpfs 4 zusammen. Der in der Aufnahme 31 ausgebildete Konus besitzt einen mittleren Konusdurchmesser d zwischen 5 und 8 mm und einen ganzen Konuswinkel oder Öffnungswinkel α zwischen 3° und 5°. Die mittlere Wandstärke s des Stumpfs 4 im Bereich der konischen Fläche 6 beträgt zwischen 0,3 und 2 mm.

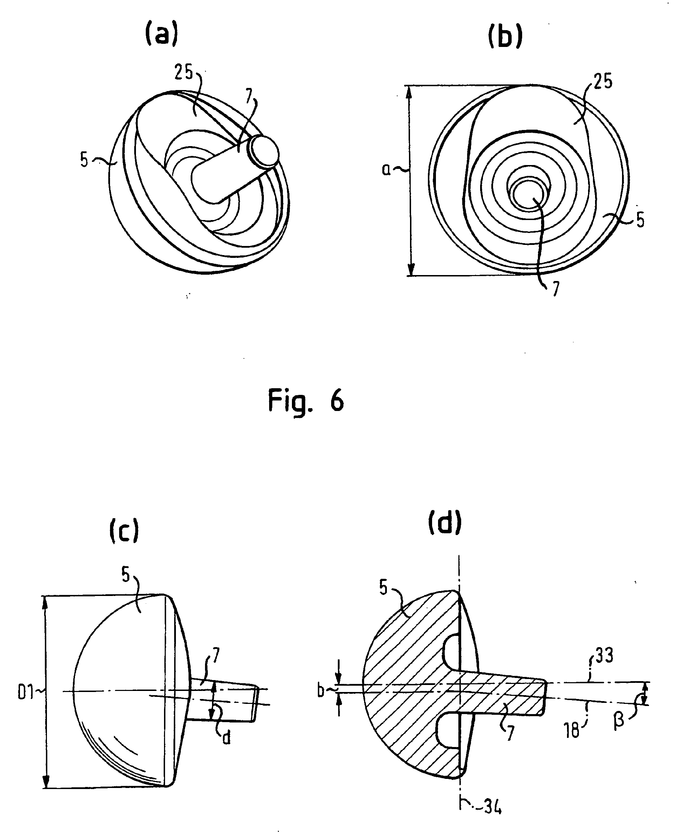

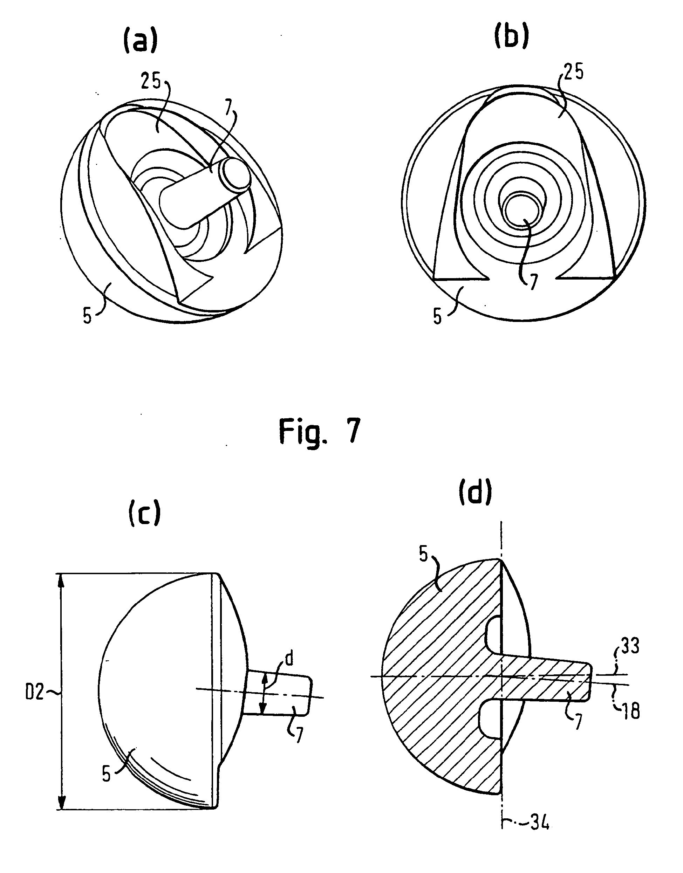

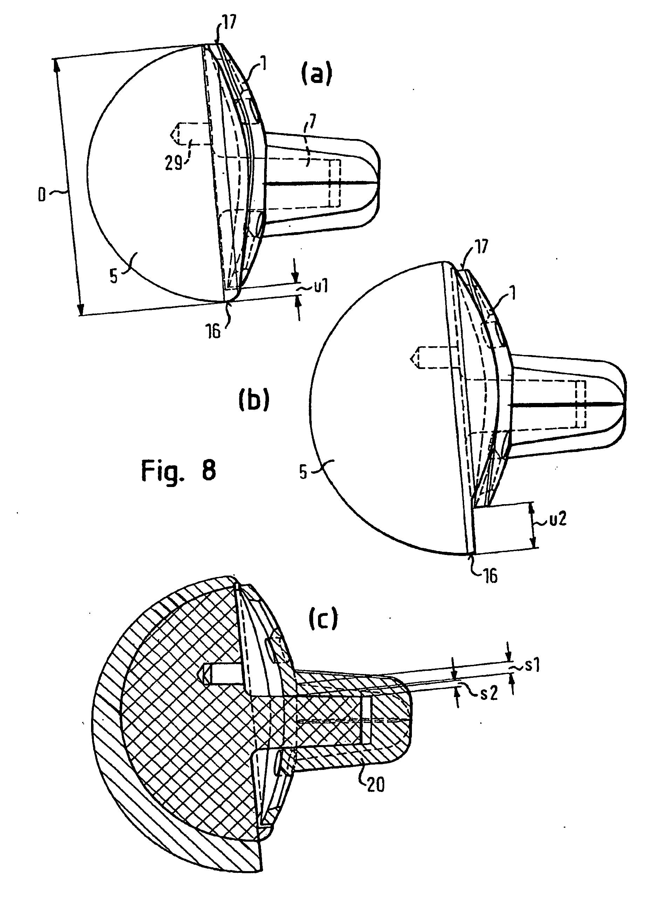

Der Stumpf 4 ist zur Befestigung eines halbkugelförmigen Gelenkteils 5 ausgelegt, wie es in den Fig. 6 und 7 in zwei verschiedenen Größen dargestellt ist. Hierzu wird ein konischer Zapfen 7 des Gelenkteils 5 mit der konischen Fläche 6 der Aufnahme 31 des Stumpfs 4 nach Art eines Press-Sitzes, wie er in Fig. 8 dargestellt ist, verbunden, so dass eine erfindungsgemäße Gelenkverbindung ausgebildet ist. Das halbkugelförmige Gelenkteil 5 ist aus einer Cr-Mo-Legierung gefertigt und kann mit einer nicht dargestellten, am Humerus befestigbaren kugelförmigen Lagerschale, die ebenfalls aus einer Cr-Mo-Legierung gefertigt ist, gepaart werden. Eine derartige Gelenkverbindung entspricht einer inversen Schulterendoprothese.

Der mittlere Konusdurchmesser und der ganze Konuswinkel des Zapfens 7 entsprechen dem Konusdurchmesser d und dem ganzen Konuswinkel α der Aufnahme 31. Die Längsachse des Zapfens 7 fällt im zusammengesetzten Zustand der Gelenkverbindung mit der Längsachse 18 des Stumpfs 4 zusammen. In Analogie zur Längsachse des Stumpfs 4 der Glenoidverankerung ist die Längsachse des Zapfens 7 des Gelenkteils 5 gegenüber einer Polachse 33 des halbkugelförmigen Gelenkteils 5 um den Winkel β nach unten geneigt und ihr Schnittpunkt mit einer Basisebene 34 des halbkugelförmigen Gelenkteils 5 um den Betrag b nach unten versetzt.

Das halbkugelförmige Gelenkteil 5 umfasst eine Ausnehmung 25, deren Kontur der Außenkontur der ovalen Platte 1 entspricht, so dass die Platte 1 beim Zusammensetzen der Gelenkverbindung passgenau in der Ausnehmung 25 des halbkugelförmigen Gelenkteils 5 versenkbar ist. Aufgrund der ovalen Form der Platte 1, deren in Längsrichtung 14 gelegenen Enden nicht symmetrisch ausgebildet sind, kann lediglich genau eine Winkellage der ovalen Platte 1 zu dem halbkugelförmigen Gelenkteil 5 erreicht werden.

Das in Fig. 6 dargestellte halbkugelförmige Gelenkteil 5 mit einem ersten Kugeldurchmesser D 1 schließt im zusammengesetzten Zustand der Gelenkverbindung gemäß Fig. 8a an der oberen Kante 17 der Platte 1 bündig mit dieser ab und steht über die untere Kante 16 der Platte 1 unter Ausbildung eines Überstands u 1 geringfügig vor. Das Gelenkteil 5 weist eine Hilfsbohrung 29 auf, die zusammen mit dem Zapfen 7 zur Halterung des Gelenkteils 5 beim Bearbeiten der Kugelfläche bei der Herstellung des Gelenkteils 5 dient.

Der zusammengesetzte Zustand einer Gelenkverbindung mit einem in Fig. 7 gezeigten halbkugelförmigen Gelenkteil 5 mit einem zweiten Kugeldurchmesser D2, der größer ist als der Kugeldurchmesser D1, ist in Fig. 8b dargestellt. Das Gelenkteil 5 von Fig. 7 steht über die obere Kante 17 der Platte 1 geringfügig und über die untere Kante 16 der Platte 1 unter Ausbildung eines Überstands u2 weiter hervor als über die obere Kante 17. Eine Überlagerung der zusammengesetzten Gelenkverbindungen aus den Fig. 8a und 8b ist in Fig. 8c dargestellt, wobei Fig. 8c insbesondere verschiedene Wandstärken s 1 und s2 des Stumpfs 4 veranschaulicht.

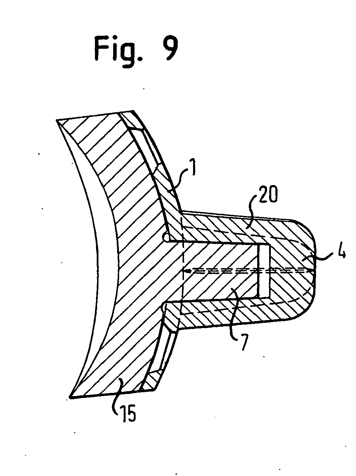

Fig. 9 zeigt ein weiteres Ausführungsbeispiel einer Gelenkverbindung, wobei jedoch lediglich die Abweichungen dieses Ausführungsbeispiels gegenüber dem in den Fig. 1 bis 8 dargestellten Ausführungsbeispiel erläutert werden sollen. Gleiche oder entsprechende Teile werden dabei mit denselben Bezugszeichen bezeichnet.

Im Gegensatz zu Fig. 8 ist in Fig. 9 eine Gelenkverbindung dargestellt, die anstelle eines halbkugelförmigen Gelenkteils ein schalenförmiges Gelenkteil 15 aufweist, in das ein nicht dargestellter, am Humerus angebrachter Gelenkkopf eingreifen kann, so dass eine konventionelle Schulterendoprothese ausgebildet ist. Das aus Polyethylen gefertigte schalenförmige Gelenkteil 15 weist dabei eine Außenkontur auf, die der Außenkontur der ovalen Platte 1 entspricht, so dass das schalenförmige Gelenkteil 15 nicht über die ovale Platte 1 vorsteht.

Nachfolgend wird das Verankern der Glenoidverankerugn im Glenoidknochen und das Zusammensetzen der erfindungsgemäßen Gelenkverbindung unter Bezugnahme auf Fig. 1 bis 9 beschrieben.

Zunächst wird der Glenoidknochen 2 mit einem sphärischen Fräser derart reseziert, dass eine der Wölbung der Platte 1 entsprechende Auflagefläche für die Platte 1 entsteht. Dann wird in die Auflagefläche mittels eines Zentralbohrers ein Loch für den Stumpf 4 der Glenoidverankerung gebohrt. Daraufhin kann die Glenoidverankerung gesetzt werden. Zur Befestigung der Glenoidverankerung werden Löcher für die Knochenschrauben 8 gebohrt, in die schließlich die Knochenschrauben 8 eingeschraubt werden. Die Glenoidverankerung ist nun fest mit dem Glenoidknochen 2 verbunden.

Danach kann das Gelenkteil 5, 15 an der Glenoidverankerung befestigt werden. Hierzu wird der Zapfen 7 des Gelenkteils 5, 15 in die Aufnahme 31 des Stumpfs 4 gedrückt und das Gelenkteil 5, 15 derart orientiert, dass die Außenkontur der Platte 1 in die Ausnehmung 25 des Gelenkteils 5, 15 eingreifen kann. Nach leichtem Andrücken wird das Gelenkteil 5, 15 dann mittels eines Einschlägers eingeschlagen. Das Gelenkteil 5, 15 ist nun über einen Press-Sitz fest mit der Glenoidverankerung verbunden.

Bei einer inversen Schulterendoprothese stehen dem Operateur verschiedene halbkugelförmige Gelenkteile 5 mit unterschiedlichen Kugeldurchmessern D zur Verfügung, aus denen der Operateur das jeweils für den Patienten passende Gelenkteil 5 auswählen kann. Die Auswahl des passenden Gelenkteils 5 kann mittels Probier-Gelenkteilen erfolgen.

Die Glenoidverankerung umfasst einen Stumpf 4, der sowohl zur Verankerung der Glenoidverankerung im Glenoidknochen 2 als auch zur Befestigung des Gelenkteils 5, 15 an der Glenoidverankerung ausgelegt ist, wobei die Befestigung des Gelenkteils 5, 15 auf besonders einfache Weise und zugleich besonders zuverlässig mittels eines Press-Sitzes realisierbar ist. Der Stumpf 4 besitzt dabei eine äußere Form, die sich als besonders geeignet für einen Press-Sitz für eine Schulterendoprothese erwiesen hat.

### Bezugszeichenliste

- 1: ovale Platte
- 2: Glenoidknochen
- 3: Bohrung
- 4: Stumpf
- 5: halbkugelförmiges Gelenkteil
- 6: konische Fläche
- 7: konischer Zapfen
- 8: Knochenschrauben
- 10: Außenfläche
- 11: Innenfläche
- 14: Längsrichtung
- 15: schalenförmiges Gelenkteil
- 16: untere Kante
- 17: obere Kante
- 18: Längsachse
- 19: Verbindungsgerade
- 20: Längsrippe
- 21: ringförmige Schneide
- 22: Durchbruch
- 25: Ausnehmung
- 29: Hilfsbohrung
- 31: Aufnahme
- 32: Senkrechte
- 33: Polachse
- 34: Basisebene
- a: Abstand
- b: Betrag
- d: Konusdurchmesser
- D: Kugeldurchmesser
- r: Radius
- s: Wandstärke
- u: Überstand

- α: Öffnungswinkel
- β: Neigungswinkel

## Patentansprüche

1. Gelenkverbindung für eine Schulterendoprothese mit einer Glenoidverankerung für ein Gelenkteil (5) und einem an der Glenoidverankerung befestigbaren, halbkugelförmigen Gelenkteil (5), wobei die Glenoidverankerung eine am resezierten Glenoidknochen (2) aufsetzbare Platte (1), die Bohrungen (3) für Befestigungselemente (8) zur Verankerung im Glenoidknochen (2) aufweist, und einen Stumpf (4) zur Befestigung des Gelenkteils (5) umfasst, wobei der Stumpf (4) mit der Platte (1) fest verbunden ist
**dadurch gekennzeichnet,**
**dass** der Stumpf (4) auf seiner Innenseite eine Aufnahme (31) mit einer konischen Fläche (6) für einen Press-Sitz mit einem konischen Zapfen (7) des Gelenkteils (5) aufweist, und
**dass** das Gelenkteil (5) mit einer oberen Kante (17) der Platte (1) bündig abschließt oder nur geringfügig über diese vorsteht und über eine untere Kante (16) der Platte (1) weiter vorsteht als über die obere Kante (17).

2. Gelenkverbindung nach Anspruch 1,
**dadurch gekennzeichnet,**
**dass** die Aufnahme (31) einen mittleren Konusdurchmesser (d) zwischen 5 und 8 mm aufweist.

3. Gelenkverbindung nach Anspruch 1 oder 2,
**dadurch gekennzeichnet,**
**dass** die Aufnahme (31) einen ganzen Konuswinkel (α) zwischen 3° und 5° besitzt.

4. Gelenkverbindung nach einem der Ansprüche 1 bis 3,
**dadurch gekennzeichnet,**
**dass** die mittlere Wandstärke (s) des Stumpfes (4) im Bereich der konischen Fläche (6) zwischen 0,3 und 2 mm liegt.

5. Gelenkverbindung nach einem der Ansprüche 1 bis 4,
**dadurch gekennzeichnet,**
**dass** die Platte (1) zum Glenoidknochen (2) hin gewölbt ist.

6. Gelenkverbindung nach Anspruch 5,
**dadurch gekennzeichnet,**
**dass** die Platte (1) mit einer Außenfläche (10) und mit einer Innenfläche (11) versehen ist, welche Ausschnitte von Kugelflächen mit einem Radius (r1, r2) zwischen 22 und 40 mm sind.

7. Gelenkverbindung nach einem der Ansprüche 1 bis 6,
**dadurch gekennzeichnet,**
**dass** bei einer ovalen Platte (1) die in Längsrichtung (14) abschließenden Kanten (16, 17) der Platte (1) einen Abstand (a) zwischen 25 und 50 mm aufweisen.

8. Gelenkverbindung nach einem der Ansprüche 1 bis 7,
**dadurch gekennzeichnet,**
**dass** der Stumpf (4) exzentrisch an der Platte (1) angebracht ist.

9. Gelenkverbindung nach einem der Ansprüche 1 bis 8,
**dadurch gekennzeichnet,**
**dass** der Schnittpunkt der Längsachse (18) des Stumpfs (4) mit einer Verbindungsgeraden (19) von Kanten (16, 17) der Platte (1) um einen Betrag (b) von 2 bis 8 mm von der Mitte der Verbindungsgeraden (19) zu einer unteren Kante (16) hin verschoben ist.

10. Gelenkverbindung nach einem der Ansprüche 1 bis 9,
**dadurch gekennzeichnet,**
**dass** die Längsachse (18) des Stumpfs (4) gegenüber einer Senkrechten (32) zu einer Verbindungsgeraden (19) von Kanten (16, 17) der Platte (1) um einen Winkel (β) von 3° bis 7° nach unten geneigt ist.

11. Gelenkverbindung nach einem der Ansprüche 1 bis 10,
**dadurch gekennzeichnet,**
**dass** die Längsachse (18) der konischen Fläche (6) mit der Längsachse (18) des Stumpfs (4) zusammenfällt.

12. Gelenkverbindung nach einem der Ansprüche 1 bis 11,
**dadurch gekennzeichnet,**
**dass** der Stumpf (4) zur drehsicheren Verankerung an seiner Außenseite mehrere insbesondere messerartige Längsrippen (20) aufweist.

13. Gelenkverbindung nach einem der Ansprüche 1 bis 12,
**dadurch gekennzeichnet,**
**dass** von der Platte (1) zum Glenoidknochen (2) hin mehrere insbesondere ringförmige Schneiden (21) mit Durchbrüchen (22) für das Einwachsen von Knochenmaterial vorstehen.

14. Gelenkverbindung nach einem der Ansprüche 1 bis 13,
**dadurch gekennzeichnet,**
**dass** verschiedene halbkugelförmige Gelenkteile (5) mit unterschiedlichen Kugeldurchmessern (D) vorgesehen sind.

15. Gelenkverbindung nach einem der Ansprüche 1 bis 14,
**dadurch gekennzeichnet,**
**dass** die Platte (1) in einer der von einer Kreisform abweichenden, insbesondere ovalen Außenkontur der Platte (1) entsprechenden Ausnehmung (25) des halbkugelförmigen Gelenkteils (5) versenkbar ist.

16. Gelenkverbindung nach einem der Ansprüche 1 bis 15,
**dadurch gekennzeichnet,**
**dass** die Platte (1) und der Stumpf (4) aus Titan oder einer Titanlegierung und das halbkugelförmige Gelenkteil (5) aus einem verschleißfesten Werkstoff, beispielsweise aus einer Cr-Mo-Legierung besteht.

17. Gelenkverbindung nach einem der Ansprüche 1 bis 16,
**dadurch gekennzeichnet,**
**dass** das halbkugelförmige Gelenkteil (5) aus einer Cr-Mo-Legierung besteht und mit einer am Humerus befestigbaren kugelförmigen Lagerschale, die aus einer Cr-Mo-Legierung besteht, gepaart ist.

18. Gelenkverbindung nach einem der Ansprüche 1 bis 17,
**dadurch gekennzeichnet,**
**dass** das halbkugelförmige Gelenkteil (5) aus einer Cr-Mo-Legierung oder aus Keramik besteht und mit einer am Humerus befestigbaren kugelförmigen Lagerschale aus Kunststoff, beispielsweise aus Polyethylen gepaart ist.

## Claims

1. A joint connection for a shoulder endoprothesis having a glenoid fixation for a joint part (5) and a hemispherical joint part (5) attachable at the glenoid fixation, the glenoid fixation including a plate (1) placeable at the resected glenoid bone (2), the plate having bores (3) for fastener elements (8) for the fixation in the glenoid bone (2), and also including a stump (4) for the fixation of the joint part (5), the stump (4) being fixedly connected to the plate (1),
**characterized in that**
the stump (4) has a mount (31) at its inner side having a conical surface (6) for a press fit with a conical spigot (7) of the joint part (5) and
**in that** the joint part (5) finishes flush with an upper edge (17) of the plate (1) or only projects slightly beyond this and projects further beyond a lower edge (16) of the plate (1) than beyond the upper edge (17).

2. A joint connection in accordance with claim 1,
**characterized in that**
the mount (31) has a mean diameter (d) of between 5 and 8 mm.

3. A joint connection in accordance with claim 1 or claim 2,
**characterized in that**
the mount (31) has a complete cone angle (α) of between 3° and 5°.

4. A joint connection in accordance with any one of claims 1 to 3,
**characterized in that**
the mean wall thickness (s) of the stump (4) in the region of the conical surface (6) is between 0.3 and 2 mm.

5. A joint connection in accordance with any one of claims 1 to 4,
**characterized in that**
the plate (1) is arced towards the glenoid bone (2).

6. A joint connection in accordance with claim 5,
**characterized in that**
the plate (1) is provided with an outer surface (10) and with an inner surface (11) which are sections of spherical surfaces having a radius (r1, r2) between 22 and 40 mm.

7. A joint connection in accordance with any one of claims 1 to 6,
**characterized in that**
for an oval plate (1) the edges (16, 17) finishing the plate (1) in the longitudinal direction (14) have a separation (a) of between 25 and 50 mm.

8. A joint connection in accordance with any one of claims 1 to 7,
**characterized in that**
the stump (4) is eccentrically attached at the plate (1).

9. A joint connection in accordance with any one of claims 1 to 8,
**characterized in that**
the point of intersection of the longitudinal axis (18) of the stump (4) with a connection straight (19) of edges (16,17) of the plate (1) is displaced from the middle of the connection straight (19) towards the lower edge by a quantity (b) of 2 to 8 mm.

10. A joint connection in accordance with any one of claims 1 to 9,
**characterized in that**
the longitudinal axis (18) of the stump (4) is tilted downwards with regard to a perpendicular (32) towards a connection straight (19) of edges (16, 17) of the plate (1) by an angle (β) of 3° to 7°.

11. A joint connection in accordance with any one of claims 1 to 10,
**characterized in that**
the longitudinal axis (18) of the conical surface (6) coincides with the longitudinal axis (18) of the stump (4).

12. A joint connection in accordance with any one of claims 1 to 11,
**characterized in that**
the stump (4) has a plurality of, in particular, knife-like longitudinal ribs (20) at its outer side for a rotationally fixed fixation.

13. A joint connection in accordance with any one of claims 1 to 12,
**characterized in that**
a plurality of, in particular, ring shaped cutting edges (21) with breakthroughs (22) for the ingrowth of bone material project from the plate (1) towards the glenoid bone (2).

14. A joint connection in accordance with any one of claims 1 to 13,
**characterized in that**
different hemispherical joint parts (5) with different sphere diameters (D) are provided.

15. A joint connection in accordance with any one of claims 1 to 14,
**characterized in that**
the plate (1) can be sunk into a recess (25) of the hemispherical joint part (5) which recess corresponds to the outer contour of the plate (1) which deviates from a circular shape and in particular corresponds to an oval outer contour of the plate (1).

16. A joint connection in accordance with any one of claims 1 to 15,
**characterized in that**
the plate (1) and the stump (4) are made from titanium or a titanium alloy and the hemispherical joint part (5) is made from a wear resistant material, for example from a Cr-Mo-alloy.

17. A joint connection in accordance with any one of claims 1 to 16,
**characterized in that**
the hemispherical joint part (5) is made from a Cr-Mo alloy and is paired with a spherical shaped bearing shell made of a Cr-Mo alloy and attachable at the humerus.

18. A joint connection in accordance with any one of claims 1 to 17,
**characterized in that**
the hemispherical joint part (5) is made from a Cr-Mo alloy or from ceramic and is paired with a spherical shaped bearing shell made of plastic, for example of polyethylene, and attachable at the humerus.

## Revendications

1. Liaison articulaire pour une endoprothèse d'épaule comprenant un ancrage glénoïde pour une partie d'articulation (5), et une partie d'articulation (5) en forme de demi-sphère susceptible d'être fixée sur l'ancrage glénoïde, dans laquelle l'ancrage glénoïde comprend une plaque (1), à poser sur l'os glénoïde (2) réséqué, qui comporte des perçages (3) pour des éléments de fixation (8) destinés à l'ancrage dans l'os glénoïde (2), et un moignon (4) destiné à la fixation de la partie d'articulation (5), le moignon (4) étant fermement relié à la plaque (1),
**caractérisée en ce que**
le moignon (4) comporte sur son côté intérieur un logement (31) avec une surface conique (6) pour établir un engagement à la presse avec un tenon conique (7) de la partie d'articulation (5), et
**en ce que** la partie d'articulation (5) se termine en affleurement avec une arête supérieure (17) de la plaque (1), ou bien ne dépasse que légèrement au-delà de celle-ci, et dépasse au-delà d'une arête inférieure (16) de la plaque (1) plus loin qu'au-delà de l'arête supérieure (17).

2. Liaison articulaire selon la revendication 1,
**caractérisée en ce que** le logement (31) présente un diamètre conique moyen (d) entre 5 et 8 mm.

3. Liaison articulaire selon la revendication 1 ou 2,
**caractérisée en ce que** le logement (31) possède un angle conique total (α) entre 3° et 5°.

4. Liaison articulaire selon l'une des revendications 1 à 3,
**caractérisée en ce que** l'épaisseur de paroi moyenne (s) du moignon (4) dans la zone de la surface conique (6) est entre 0,3 et 2 mm.

5. Liaison articulaire selon l'une des revendications 1 à 4,
**caractérisée en ce que** la plaque (1) est bombée en direction de l'os glénoïde (2).

6. Liaison articulaire selon la revendication 5,
**caractérisée en ce que** la plaque (1) est dotée d'une surface extérieure (10) et d'une surface intérieure (11) qui sont des segments de surfaces sphériques avec un rayon (r1, r2) entre 22 et 40 mm.

7. Liaison articulaire selon l'une des revendications 1 à 6,
**caractérisée en ce que**, pour une plaque ovale (1), les arêtes terminales (16, 17) en direction longitudinale (14) de la plaque (1) présentent une distance (a) entre 25 et 50 mm.

8. Liaison articulaire selon l'une des revendications 1 à 7,
**caractérisée en ce que** le moignon (4) est agencé excentriquement sur la plaque (1).

9. Liaison articulaire selon l'une des revendications 1 à 8,
**caractérisée en ce que** le point d'intersection de l'axe longitudinal (18) du moignon (4) avec une droite de jonction (19) des arêtes (16, 17) de la plaque (1) est déporté d'une distance (b) de 2 à 8 mm depuis le centre de la droite de jonction (19) en direction d'une arête inférieure (16).

10. Liaison articulaire selon l'une des revendications 1 à 9,
**caractérisée en ce que** l'axe longitudinal (18) du moignon (4) est incliné vers le bas, d'un angle (β) de 3° à 7° par rapport à une perpendiculaire (32) à une ligne de jonction (19) des arêtes (16, 17) de la plaque (1).

11. Liaison articulaire selon l'une des revendications 1 à 10,
**caractérisée en ce que** l'axe longitudinal (18) de la surface conique (6) coïncide avec l'axe longitudinal (18) du moignon (4).

12. Liaison articulaire selon l'une des revendications 1 à 11,
**caractérisée en ce que** le moignon (4) comporte sur sa face extérieure plusieurs nervures longitudinales (20), en particulier semblables à des couteaux, pour un ancrage bloqué en rotation.

13. Liaison articulaire selon l'une des revendications 1 à 12, **caractérisée en ce que** plusieurs tranchants (21), en particulier de forme annulaire, avec des traversées (22) pour la croissance de matériau osseux, dépassent de la plaque (1) en direction de l'os glénoïde (2).

14. Liaison articulaire selon l'une des revendications 1 à 13, **caractérisée en ce qu'**il est prévu différentes parties d'articulation (5) en forme de demi-sphère avec des diamètres de sphère (D) différents.

15. Liaison articulaire selon l'une des revendications 1 à 14, **caractérisée en ce que** la plaque (1) est susceptible d'être noyée dans un évidement (25), de la partie d'articulation (5) en forme de demi-sphère, qui correspond au contour extérieur de la plaque (1), différent d'une forme circulaire, en particulier ovale.

16. Liaison articulaire selon l'une des revendications 1 à 15, **caractérisée en ce que** la plaque (1) et le moignon (4) sont en titane ou un alliage de titane, et la partie d'articulation (5) en forme de demi-sphère (5) est en un matériau résistant à l'usure, par exemple un alliage Cr-Mo.

17. Liaison articulaire selon l'une des revendications 1 à 16, **caractérisée en ce que** la partie d'articulation (5) en forme de demi-sphère est en un alliage Cr-Mo, et est appariée à une coque de montage de forme sphérique, susceptible d'être fixée sur l'humérus, constituée en un alliage Cr-Mo.

18. Liaison articulaire selon l'une des revendications 1 à 17, **caractérisée en ce que** la partie d'articulation (5) en forme de demi-sphère est en un alliage Cr-Mo ou en céramique, et est appariée à une coque de montage de forme sphérique, susceptible d'être fixée sur l'humérus, en matière plastique, par exemple en polyéthylène.
